# EUROPEAN PATENT SPECIFICATION

(11) **EP 3 149 165 B1**
(45) Date of publication and mention of the grant of the patent: **22.04.2020**
(21) Application number: 15725008.5
(22) Date of filing: 26.05.2015
(51) Int. Cl.: C12N 9/88, C12P 7/10

(54) **CAUSATIVE GENES CONFERRING ACETIC ACID TOLERANCE IN YEAST**
KAUSATIVE GENE ZUR INDUZIERUNG VON ESSIGSÄURETOLERANZ BEI HEFE
GÈNES CAUSAUX CONFÉRANT UNE TOLÉRANCE À L'ACIDE ACÉTIQUE CHEZ LA LEVURE

(30) Priority: 26.05.2014 EP 14169848
(43) Date of publication of application: 05.04.2017
(73) Proprietor: VIB VZW, 9052 Gent (BE); Katholieke Universiteit Leuven K.U. Leuven R&D, 3000 Leuven (BE)
(72) Inventor: THEVELEIN, Johan, B-3052 Blanden (BE); FOULQUIÉ MORENO, Maria Remedios, B-1040 Brussel (BE); MEIJNEN, Jean-Paul, 3911 AW Rhenen (NL)
(74) Representative: Demolder, Jan Albert
(86) International application number: PCT/EP2015/061590
(87) International publication number: WO 2015/181169

(56) References cited:
- WO-A1-2014/053648
- "UNIPROT:C8ZEK3", , 3 November 2009 (2009-11-03), XP055202858, Retrieved from the Internet: URL:http://ibis.internal.epo.org/exam/dbfe tch.jsp?id=UNIPROT:C8ZEK3 [retrieved on 2015-07-16] -& NOVO M ET AL: "Eukaryote-to-eukaryote gene transfer events revealed by the genome sequence of the wine yeast Saccharomyces cerevisiae EC1118", PROCEEDINGS OF THE NATIONAL ACADEMY OF SCIENCES, vol. 106, no. 38, 22 September 2009 (2009-09-22), pages 16333-16338, XP055187194, ISSN: 0027-8424, DOI: 10.1073/pnas.0904673106
- "UNIPROT:B5VPC4", , 25 November 2008 (2008-11-25), XP055202862, Retrieved from the Internet: URL:http://ibis.internal.epo.org/exam/dbfe tch.jsp?id=UNIPROT:B5VPC4 [retrieved on 2015-07-16] -& BORNEMAN A R ET AL: "Comparative genome analysis of a Saccharomyces cerevisiae wine strain", FEMS YEAST RESEARCH, vol. 8, no. 7, 5 September 2008 (2008-09-05), pages 1185-1195, XP055202870, ISSN: 1567-1356, DOI: 10.1111/j.1567-1364.2008.00434.x
- "UNIPROT:B3LLP8", , 2 September 2008 (2008-09-02), XP055202873, Retrieved from the Internet: URL:http://ibis.internal.epo.org/exam/dbfe tch.jsp?id=UNIPROT:B3LLP8 [retrieved on 2015-07-16]
- GONZALEZ-RAMOS D ET AL: "A Recombinant Saccharomyces cerevisiae Strain Overproducing Mannoproteins Stabilizes Wine against Protein Haze", APPLIED AND ENVIRONMENTAL MICROBIOLOGY, vol. 74, no. 17, 7 July 2008 (2008-07-07), pages 5533-5540, XP055203255, ISSN: 0099-2240, DOI: 10.1128/AEM.00302-08
- CEBOLLERO E ET AL: "Construction of a recombinant autolytic wine yeast strain overexpressing the csc1-1 allele", BIOTECHNOLOGY PROGRESS, 1 September 2009 (2009-09-01), pages 1598-1604, XP055203253, ISSN: 8756-7938, DOI: 10.1002/btpr.269
- PERLSTEIN E O ET AL: "Revealing Complex Traits with Small Molecules and Naturally Recombinant Yeast Strains", CHEMISTRY AND BIOLOGY, vol. 13, no. 3, 1 March 2006 (2006-03-01), pages 319-327, XP027991230, ISSN: 1074-5521
- MIRA N P ET AL: "Adaptive Response and Tolerance to Weak Acids in Saccharomyces cerevisiae : A Genome-Wide View", OMICS: A JOURNAL OF INTEGRATIVE BIOLOGY, vol. 14, no. 5, 1 October 2010 (2010-10-01), pages 525-540, XP055202971, ISSN: 1536-2310, DOI: 10.1089/omi.2010.0072 cited in the application
- MIRA N P ET AL: "Genome-wide identification of Saccharomyces cerevisiae genes required for tolerance to acetic acid", MICROBIAL CELL FACTORIES, vol. 9, no. 1, 25 October 2010 (2010-10-25), page 79, XP021077227, ISSN: 1475-2859, DOI: 10.1186/1475-2859-9-79 cited in the application
- HASUNUMA T ET AL: "Metabolic pathway engineering based on metabolomics confers acetic and formic acid tolerance to a recombinant xylose-fermenting strain of Saccharomyces cerevisiae", MICROBIAL CELL FACTORIES, vol. 10:2, 10 January 2011 (2011-01-10), pages 1-13, XP021088153, ISSN: 1475-2859, DOI: 10.1186/1475-2859-10-2 cited in the application
- HAITANI Y ET AL: "Identification of an acetate-tolerant strain of Saccharomyces cerevisiae and characterization by gene expression analysis", JOURNAL OF BIOSCIENCE AND BIOENGINEERING, vol. 114, no. 6, 27 July 2012 (2012-07-27) , pages 648-651, XP055202920, ISSN: 1389-1723, DOI: 10.1016/j.jbiosc.2012.07.002
- PEÑA P V ET AL: "Genome-wide overexpression screen for sodium acetate resistance inSaccharomyces cerevisiae", JOURNAL OF BIOTECHNOLOGY, vol. 164, no. 1, 20 December 2012 (2012-12-20), pages 26-33, XP028986659, ISSN: 0168-1656, DOI: 10.1016/J.JBIOTEC.2012.12.005
- KIMURA A ET AL: "Glyoxylase I in microorganisms: Molecular characteristics, genetics and biochemical regulation", BIOCHEMICAL SOCIETY TRANSACTIONS; 645TH MEETING OF THE BIOCHEMICAL SOCIETY ON PHOSPHOLIPID TRANSLOCATION, ASYMMETRY AND MEMBRANE FUSION, vol. 21, no. 2, 1 May 1993 (1993-05-01), pages 518-521, XP008159959, ISSN: 0300-5127, DOI: 10.1042/BST0210518
- INOUE Y ET AL: "Glyoxalase system in yeasts: Structure, function, and physiology", SEMINARS IN CELL & DEVELOPMENTAL BIOLOGY, vol. 22, no. 3, 1 May 2011 (2011-05-01), pages 278-284, XP055202949, ISSN: 1084-9521, DOI: 10.1016/j.semcdb.2011.02.002
- KARAGÖZ P ET AL: "Ethanol production from wheat straw by Saccharomyces cerevisiae and Scheffersomyces stipitis co-culture in batch and continuous system", BIORESOURCE TECHNOLOGY, vol. 158, 15 February 2014 (2014-02-15), pages 286-293, XP055408253, GB ISSN: 0960-8524, DOI: 10.1016/j.biortech.2014.02.022
- GUTIÉRREZ-RIVERA B ET AL: "Conversion efficiency of glucose/xylose mixtures for ethanol production using Saccharomyces cerevisiae ITV01 and Pichia stipitis NRRL Y-7124", JOURNAL OF CHEMICAL TECHNOLOGY AND BIOTECHNOLOGY, vol. 87, no. 2, 10 August 2011 (2011-08-10), pages 263-270, XP055408264, ISSN: 0268-2575, DOI: 10.1002/jctb.2709
- ASHOOR S ET AL: "Cell-recycle batch process of Scheffersomyces stipitis and Saccharomyces cerevisiae co-culture for second generation bioethanol production", BIOTECHNOLOGY LETTERS, vol. 37, no. 11, 22 July 2015 (2015-07-22) , pages 2213-2218, XP035538829, ISSN: 0141-5492, DOI: 10.1007/S10529-015-1919-9 [retrieved on 2015-07-22]
- Soo Rin Kim ET AL: "Strain engineering of Saccharomyces cerevisiae for enhanced xylose metabolism", BIOTECHNOLOGY ADVANCES., vol. 31, no. 6, 1 November 2013 (2013-11-01), pages 851-861, XP055575571, GB ISSN: 0734-9750, DOI: 10.1016/j.biotechadv.2013.03.004

SEQUENCE LISTING

<110>  VIB VZW
       KATHOLIEKE UNIVERSITEIT LEUVEN, K.U.LEUVEN R&D

<120>  Causative genes conferring acetic acid tolerance in yeast

<130>  JT/GLO1/494

<150>  EP 14169848.0
<151>  2014-05-26

<160>  2

<170>  PatentIn version 3.5

<210>  1
<211>  2745
<212>  DNA
<213>  Saccharomyces cerevisiae

<400>  1
tcactgaaaa atatttattt tttcagtgtc tacttcgtgg cctttgaaat gtgtagtaag      60

cctagaccat atatcatcgt aaagatttag ttcaaaatag tcgtgtataa gatcgtaaag     120

attagaaaag ttcttgaaaa gtgaatccga tttcattcca tcaaatgctt gttgcacgat     180

gctttcaaaa atacttctag atggatcatc accaggagcg agcgttctgt aactcgaaaa     240

caattggcca agctgtgtta atcgagcagg atgaatgtgc aatatattct cgaatgacga     300

tctttcttgc tgaaaagaat ctatcgacag atttggcata gcatgtgcat tttttggtat     360

tttctcataa ttgtgaaaac actgtaagta gtcgttaggt acaaattcgc aatttaaagg     420

ttgatctatg aatagtatga taaaatcagc cgccccttca aagtagtcat taaaattggg     480

taatattttt gtctctagaa tctttagtct tcgccttata tgaaatccat ttaaggcatg     540

gatcacatta ttcttccatt gaataacaac ggtcttacca tctaacgttt cataatgact     600

gtcccttgaa ttcgtattta tgtaagagtt ttcagtcatt aacaatatgt aagagttaat     660

aaactctgca ttataacaga ggtcatgtaa cggcagtttg ctcttgacat cgtggtaatt     720

caaaaggtat tctgaaggag gtgctaggag gataaaccta cgggttttta aattagcgaa     780

aagctttttc aatggggatc tttctggttc gggacagtta aatatagcat taaccagcgg     840

gttcaacagc gttggtaaat ggtaaactga cattgtcaac cctagctaag ccatagcttt     900

tccgttgagg aatttacaat aaggtggttc ctttagttat aaattgcaac tgccaaaatt     960

ttcgggtagc ggcaaaatta aacgaaaacc ttctgtcata ttaaacatag tttagtgacg    1020

caatgcacac acactcatat atatgtatat atttatctat atcataggtg taaggcaaag    1080

ttatcacgtg aatatactgt ttagtataca aatacgttga tttaacggct gagagattcc    1140

caaacaaagt aacgcttagt ttagctaaag tagaatcgaa gcgcagtgaa ggcaagacga    1200

gttatccctt tatcacaaaa tataaaaaat aggtaaagag gggggtgggg gtggttcgga    1260

ctttgcatct cggtgcatta gtaccactca gcttcagtta taggaatact gacaatgttc 1320

ttgagccagg gagaactgaa atttccatac cctttaaccc tttattcttg agcatatgat 1380

cttatttttc cactcagtac aaaatacaca tcccgcatat gatctggaca ctgaataaac 1440

taggggcttt atgatggagt agtagacctg gatacgtagt caccacaggg atcctaaact 1500

gcgtcatagt aagtttcttt gatactagaa tgtccgtctc tcgtagtagt tgatatcccg 1560

tatttctagt tagtgacgtt tataaatagg gagaaaaaaa atcggagtaa catttccatg 1620

cctttttttg acacttcaga ccagatacgc caccagctac aaactaacaa tgtccactga 1680

tagtacacgc tatccaattc agattgagaa agcctcgaat gatccaaccc ttctgcttaa 1740

tcacacatgt ttaagagtca aggatccagc aagggctgtt aagttctaca ccgaacactt 1800

cggtatgaag ctattaagca gaaaggattt tgaagaagca aaatttagct tgtacttttt 1860

aagctttcca aaagacgaca tacccaaaaa taagaatgga gagcctgatg tttttagcgc 1920

acacggtgtc ttagaactaa ctcacaattg gggtactgaa aaaaacccag actacaagat 1980

caacaacggg aatgaggaac ctcatcgtgg atttgggcac atctgttttt ctgtatccga 2040

tatcaataaa acctgcgaag agctagaatc tcagggtgtc aaattcaaga agagactctc 2100

tgaaggaaga cagaaggaca ttgcgtttgc tttagaccct gatggatact ggattgagtt 2160

gatcacatat tctagagagg gtcaggaata cccaaagggc tcagtaggta acaagttcaa 2220

tcataccatg attcgtatta aaaacccaac ccggtcttta gaattctacc agaatgtgtt 2280

gggaatgaaa ttattaagaa ctagtgagca cgaaagtgca aaatttacgt tatactttct 2340

tggttatggc gttccaaaga ccgacagcgt tttttcatgt gaaagtgtgt tggagttaac 2400

tcataattgg ggaactgaga atgatccaaa cttccactat cataacggta actcagagcc 2460

ccagggttat ggtcacatct gcataagttg cgatgacgct ggcgcccttt gtaaagaaat 2520

tgaagtgaaa tacggcgata agatccaatg gtctcctaaa tttaaccaag gcagaatgaa 2580

gaatattgcc tttttgaagg atcctgatgg ttattccatt gaagtcgttc cttatggttt 2640

gattgcctaa aaagttgaaa tgaataggag gttagtattt tttttttaca ataatgacca 2700

ttcttgttga cttttcatac actaattatt atttatattt tgtat 2745

<210> 2
<211> 326
<212> PRT
<213> Saccharomyces cerevisiae

<400> 2

Met Ser Thr Asp Ser Thr Arg Tyr Pro Ile Gln Ile Glu Lys Ala Ser
1               5                   10                  15

Asn Asp Pro Thr Leu Leu Leu Asn His Thr Cys Leu Arg Val Lys Asp
            20                  25                  30

Pro Ala Arg Ala Val Lys Phe Tyr Thr Glu His Phe Gly Met Lys Leu
        35                  40                  45

Leu Ser Arg Lys Asp Phe Glu Glu Ala Lys Phe Ser Leu Tyr Phe Leu
    50                  55                  60

Ser Phe Pro Lys Asp Asp Ile Pro Lys Asn Lys Asn Gly Glu Pro Asp
65                  70                  75                  80

Val Phe Ser Ala His Gly Val Leu Glu Leu Thr His Asn Trp Gly Thr
                85                  90                  95

Glu Lys Asn Pro Asp Tyr Lys Ile Asn Asn Gly Asn Glu Glu Pro His
            100                 105                 110

Arg Gly Phe Gly His Ile Cys Phe Ser Val Ser Asp Ile Asn Lys Thr
        115                 120                 125

Cys Glu Glu Leu Glu Ser Gln Gly Val Lys Phe Lys Lys Arg Leu Ser
    130                 135                 140

Glu Gly Arg Gln Lys Asp Ile Ala Phe Ala Leu Asp Pro Asp Gly Tyr
145                 150                 155                 160

Trp Ile Glu Leu Ile Thr Tyr Ser Arg Glu Gly Gln Glu Tyr Pro Lys
                165                 170                 175

Gly Ser Val Gly Asn Lys Phe Asn His Thr Met Ile Arg Ile Lys Asn
            180                 185                 190

Pro Thr Arg Ser Leu Glu Phe Tyr Gln Asn Val Leu Gly Met Lys Leu
        195                 200                 205

Leu Arg Thr Ser Glu His Glu Ser Ala Lys Phe Thr Leu Tyr Phe Leu
    210                 215                 220

Gly Tyr Gly Val Pro Lys Thr Asp Ser Val Phe Ser Cys Glu Ser Val
225                 230                 235                 240

Leu Glu Leu Thr His Asn Trp Gly Thr Glu Asn Asp Pro Asn Phe His
                245                 250                 255

Tyr His Asn Gly Asn Ser Glu Pro Gln Gly Tyr Gly His Ile Cys Ile
            260                 265                 270

Ser Cys Asp Asp Ala Gly Ala Leu Cys Lys Glu Ile Glu Val Lys Tyr
        275                 280                 285

Gly Asp Lys Ile Gln Trp Ser Pro Lys Phe Asn Gln Gly Arg Met Lys
    290                 295                 300

Asn Ile Ala Phe Leu Lys Asp Pro Asp Gly Tyr Ser Ile Glu Val Val
305                 310                 315                 320

Pro Tyr Gly Leu Ile Ala
                325

## Description

The present invention relates to the use of *GLO1* to modulate acetic acid tolerance in yeast. More specifically, it relates to the use of a specific *GLO1* allele to confer tolerance to acetic acid, and to improve the fermentation performance of yeast in the presence of acetic acid.

Hydrolysates of lignocellulose are an interesting source for the production of bioethanol. However, one of the problems is the presence of toxic compounds such as acetic acid, furfural and lignin derivatives. Resistance against these inhibitors is essential for an efficient bioethanol production (Olsson and Hahn-Hägerdal, 1993). Especially acetic acid is known to have an inhibitory effect (Limtong et al., 2000). However, although overexpression of a single gene may improve acetic acid tolerance (Tanaka et al., 2012), it is important to understand the interplay of genes, proteins and other components that determine the physiological properties of a microorganism.

In the past, research focused indeed primarily on the identification of single alleles or genetic loci that are involved in physiological traits (Glazier et al., 2002). However, in contrast to Mendelian traits (traits that are caused by one single locus), quantitative traits are caused by multiple genetic loci, which makes the unraveling of these complex traits rather difficult (Steinmetz et al., 2002). In addition, the genetic mapping of quantitative trait loci (QTL) is hampered by genetic heterogeneity, variable phenotypic contributions of each QTL, epistasis and gene-environment interactions (Flint and Mott, 2001). These limitations have facilitated the development of novel technologies to simultaneously identify genomic loci that are involved in complex traits. With these technologies, phenotypes like high-temperature tolerance, efficient sporulation and chemical resistance have been genetically unraveled (Steinmetz et al, 2002; Deutschbauer and Davies, 2005; Ehrenreich et al., 2010).

Recently, Swinnen et al. (2012) developed such a strategy, which was successfully employed to identify genetic determinants that are involved in high ethanol tolerance in the yeast Saccharomyces cerevisiae. In this strategy, called pooled-segregant whole-genome sequence analysis, it was demonstrated that QTLs underlying a complex trait can be mapped using small populations of segregants. However, the identification of causative mutations in these QTLs remains cumbersome since this method results in a relatively large size of the identified loci, which infers the analysis of a large number of genes. Reducing the size of QTLs can be achieved with inbreeding crosses, as was recently described by Parts et al (2011). However, the use of very large pools makes it an extensive procedure, especially since phenotyping industrially relevant traits often requires elaborate procedures, making the use of large numbers of segregants undesirable. Furthermore, although inbreeding crosses can be used to decrease the size QTLs, it remains unknown how it influences the mapping of minor loci.

In order to investigate the effect of inbreeding crosses on QTL mapping of industrially relevant strains, we have applied the pooled-segregant whole-genome sequencing analysis methodology on F1 and F7 segregants of a cross between a yeast strain that is superior for acetic acid tolerance and an industrial strain that is inferior for the same trait. Acetic acid tolerance is an industrially important characteristic as yeast fermentation is severely inhibited by this weak organic acid. As mentioned above, the presence of acetic acid in lignocellulosic hydrolysate strongly affects the fermentative capacity of yeast (Casey et al., 2010; Huang et al., 2011; Narendranath et al, 2001; Taherzadeh et al., 1997; Almeida et al., 2007). Especially the fermentation of pentose sugars suffers from the presence of acetic acid (Casey et al., 2010; Bellissimi et al, 2009; Matsushika and Sawayama, 2012), emphasizing the importance of high acetic acid tolerance to enable efficient conversion of all sugars in lignocellulosic hydrolysate into ethanol. However, multiple attempts to rationally engineer increased acetic acid tolerance in yeast were met with limited success as a high number of genes is involved in the response to acetic acid stress (Abott et al., 2007; Mira et al., 2010 a & b; Li and Yuan, 2010, Hasunuma et al., 2011; Zhang et al., 2011) . Random approaches such as evolutionary engineering has rendered improved strains in terms of acetic acid tolerance (Koppram et al., 2012; Wright et al., 2011), but this method leads to overselection of a single trait and to possible loss of other important properties.

We found for the first time that increased recombination frequency indeed results in the expected smaller loci, but also in unexpected appearance and disappearance of QTLs, compared to QTL mapping without inbreeding crosses. Furthermore, combining individual whole-genome sequencing data of acetic acid tolerant segregants with bioinformatics analysis enabled QTL mapping to single gene level.

Surprisingly we found that a specific allele of *GLO1* is needed and sufficient to confer tolerance to relatively high concentrations of acetic acid. Replacement of the inferior allele by a superior allele results in a significant improvement of the fermentation performance in presence of at least 0.5% acetic acid.

A first aspect of the disclosure is the use of *GLO1* to modulate the acetic acid tolerance in yeast. Preferably, said use is the use of a specific allele of *GLO1* to increase the acetic acid tolerance, even more preferably said specific allele is encoding SED ID No.2, even more preferably said specific allele consist of SEQ ID No.1. A first aspect of the invention is a xylose fermenting yeast strain that is mutated or genetically engineered to produce ethanol on the base of xylose, comprising a GLO1 allele, which confers to the strain an improved fermentation performance in the presence of at least 0.4% acetic acid in the medium as compared to a control strain with an identical background except for the GLO1 allele, and wherein the improved fermentation performance is at least one of faster fermentation rate and a shorter lag period, wherein said GLO1 allele is an allele encoding SEQ ID NO:2 or an allele consisting of SEQ ID NO:1.

In a preferred embodiment, the use according to the disclosure is the overexpression of the protein, encoded by the specific allele. Such overexpression can be obtained by any method known to the person, skilled in the art. As a non-limiting example, overexpression can be obtained by incorporating more than one copy of the specific allele in a strain, or by placing the coding sequence of the specific allele under control of a strong promoter. In another preferred embodiment, said use according to the disclosure is the replacement of an inferior allele by the allele according to the disclosure. In a preferred embodiment of the invention is provided a xylose fermenting yeast strain as described above, wherein the GLO1 alelle is overexpressed by at least one of incorporating more than one copy of the allele in the strain, or by placing the coding sequence of the allele under control of a strong promoter.

Acetic acid tolerance as used here means that the fermentation performance of the strain in presence of acetic acid is better than that of a control strain with the same genetic background, except for the *GLO1* allele. The concentration of acetic acid in the medium is at least 0.4%, preferably at least 0.5%, more preferably at least 0.6%, even more preferably at least 0.7%, most preferably at least 0.8%. An improved fermentation performance may be measured as a higher ethanol yield, a faster fermentation rate of a shorter lag phase. Preferably said improved fermentation performance is a faster fermentation rate and/or a shorter lag period.

A GLO1 allele is called a "superior GLO1 allele" herein if, in a strain with an identical background, except for the GLO1 allele, the presence of the GLO1 allele allows improved fermentation performance in the presence of at least 0.4% acetic acid in the medium as compared to a relevant control. Analogously, a GLO1 allele is termed an "inferior GLO1 allele" herein if, in a strain with an identical background, except for the GLO1 allele, the presence of the GLO1 allele results in worse fermentation performance in the presence of at least 0.4% acetic acid in the medium as compared to a relevant control. The same applies for higher concentrations of acetic acids, e.g. 0.5%, 0.6%, 0.7%, 0.8%.

Said yeast according to the invention is a xylose fermenting yeast. A xylose fermenting yeast, as used here, is a yeast that is mutated and/or genetically engineered to ferment xylose and to produce ethanol on the base of xylose. Disclosed is such a yeast that is naturally producing ethanol on the base of xylose. Even more preferably, said yeast is selected from the group consisting of *Saccharomyces* sp., *Pichia* sp., *Candida* sp., *Pachysolen* sp. and *Spathaspora* sp. Most preferably, said yeast is a *Saccharomyces* sp. preferably a *Saccharomyces cerevisiae*.

Disclosed is a recombinant yeast strain as described above, comprising a recombinant allele encoding SEQ ID No.2. In a further aspect of the disclosure, said recombinant yeast strain comprises a recombinant allele consisting of SEQ ID No. 1.

Still another aspect of the disclosure is a method to obtain an acetic acid tolerant yeast, by crossing in a superior *GLO1* allele. Crossing in, as used here, can be by classical breeding, either by making a heterozygous diploid (comprising an inferior and a superior allele), of by mating and sporulation, selecting the strain comprising the superior allele. In a preferred embodiment, crossing in comprises the replacement of an inferior *GLO1* allele by a superior *GLO1* allele. In a preferred embodiment, said superior *GLO1* allele is encoding SEQ ID No.2. Preferably, said superior *GLO1* allele is consisting of SEQ ID No.1.

Still another aspect of the disclosure is a method for selecting acetic acid tolerant yeast, comprising the identification of the presence of a superior *GLO1* allele. Said identification can be done by any method known to the person skilled in the art. Preferably, said method comprises the sequencing of the *GLO1* allele. Preferably, said superior GLO1 allele is encoding SEQ ID No.2. Even more preferably, said superior GLO1 allele is consisting of SEQ ID No.1.

The scope of the invention is determined by the claims.

### BRIEF DESCRIPTION OF THE FIGURES

**Figure 1****:** Small scale fermentation of RHA strains for *GLO1*. The sugar conversion is expressed as % weight loss.
**Figure 2****:** Replacement of *GLO1* in ER18 by *GLO1* from 16D (A) replacement of *GLO1* in ER18 by a kanamycin marker; (B) removal of the kanamycin cassette (C) Tagging of *GLO1* in 16D by a kanamycin cassette and isolation of the *GLO1* kanamycin fragment by PCR (D) Transformation of the tagged fragment in ER18 and removal of the kanamycin cassette.
**Figure 3****:** Fermentation performance of the parental ER18 strain, compared with the ER18 variant in which the original inferior *GLO1* allele has been replaced by a superior 16D allele

### EXAMPLES

### Material and methods to the examples

### Strains used in the study

Ethanol red is a diploid industrial strain, and was obtained from Fermentis. The strain was sporulated, and the haploid segregants ER18 and 16D have been isolated on the base of the difference in their acetic acid resistance (see Table I)

**Table I**

| **Stains used for RHA analysis GLO1** | | |
|---|---|---|
| **Name** | **Genotype** | **Stocknumber** |
| ER18 | Inferior parent | JT 24050 |
| 16D | Superior parent | JT 24211 |
| ER18 x 16D | Hybrid of ER18 and 16D | JT 24198 |
| BY4741 *glo1Δ::KanMX4* | Isogenic to BY4741; except *glo1 Δ::KANMX* | JT_a.390 |
| 16D *glo1Δ::KanMX4 colony1* | Isogenic to 16D; except *glo1 Δ::KANMX* | PV_T1 |
| 16D *glo1Δ::KanMX4 colony2* | Isogenic to 16D; except *glo1 Δ::KANMX* | PV_T2 |
| ER18 *glo1Δ::KanMX4 colony1* | Isogenic to ER18; except *glo1 Δ::KANMX* | PV_T3 |
| ER18 *glo1Δ::KanMX4 colony2* | Isogenic to ER18; except *glo1 Δ::KANMX* | PV_T4 |
| ER18 x 16D *glo1Δ::KanMX4 colony1* | Hybrid of ER18 and 16D *glo1Δ::KanMX4* colony1 | PV_T5 |
| ER18 x 16D *glo1Δ::KanMX4 colony2* | Hybrid of ER18 and 16D *glo1Δ:KanMX4* colony2 | PV_T6 |
| ER18 *glo1Δ:KanMX4* x 16D colony1 | Hybrid of 16D and ER *glo1Δ:KanMX4* colony1 | PV_T7 |
| ER18 *glo1Δ:KanMX4* x 16D colony2 | Hybrid of 16D and ER *glo1Δ:KanMX4* colony2 | PV_T8 |

### Construction of RHA strains

The reciprocal deletions were engineered in the haploid strains, after which the proper haploids were crossed to obtain the diploid hybrids. The haploid deletion strains were created by gene targeting in the parental strains 16D and ER18. Deletion cassettes were PCR amplified from genomic DNA of strain BY4741 glo1Δ::KANMX4 (JT_a.390), obtained from the deletion collection (Winzeler et al., 1999), and primers B-2344 and B-2345. After transformation with the lithium acetate method (Gietz et al., 1995), transformants were selected on YPD plates containing geneticin (200mg/l). Deletion of GLO1 was confirmed by PCR with primercouple A-3863/B-2612. Of each transformed strain, two transformants were selected and subsequently crossed with the corresponding parental strain to construct the hybrid diploid strains. Mating type of the diploids was confirmed by diagnostic PCR for the MAT locus (Huxley et al., 1990).

### Assessment of acetic acid tolerance

Acetic acid tolerance in media containing acetic acid and glucose was evaluated by determination of the fermentation performance of yeast strains in small scale, near anaerobic batch fermentations. Yeast strains were pre-cultured in YPD medium (30°C, static incubation and 60 hour). After collection (1700 g, 2 minutes) and washing of the cells with Milli-Q water, cylindrical glass tubes containing 100 ml of YP medium supplemented with 4% w/v D-glucose and a range of acetic acid, adjusted to pH=4 with HCL or KOH, were inoculated at an OD600 of 0.3. The culture was agitated continuously at 120 rpm using a magnetic rod. The fermentations were performed at 30°C. The course of the fermentation was monitored by weighing the fermentation tubes at regular intervals.

### Example 1: Screening for superior acetic acid tolerance

Ethanol Red is a diploid yeast strain that is being used for bio-ethanol production at high temperatures, showing ethanol yields of up to 18 %. However, the fermentation performance of this industrial yeast strain is severely affected by acetic acid, a weak organic acid present in high quantities in lignocellulosic hydrolysates. Haploid segregants were isolated from this yeast strain and scored on acetic acid tolerance by fermentation in YPD medium supplemented with various concentrations of acetic acid. It was observed that the maximum tolerance of Ethanol Red towards acetic acid was 0.6 % (v/v) in YPD medium at a pH of 4.0. However, the lag phase was significantly prolonged by adding acetic acid to the growth medium, with a lag phase of approximately 30 hours at concentrations of 0.5 % and 0.6 %. The haploid Ethanol Red segregant #18 (named ER18) showed similar tolerance to acetic acid and was therefore selected for further experiments.

In order to obtain a yeast strain with high acetic acid tolerance, the in-house yeast collection and the yeast collection from the Fungal Biodiversity Centre (CBS-KNAW, Utrecht, The Netherlands) were screened under acetic acid conditions. More than 1000 yeast strains were assessed, from which strain JT 22689 showed the best performance under fermentative conditions at high acetic acid concentrations, being able to ferment glucose in the presence of 0.9 % acetic acid without a lag phase (not shown). Also from this strain a haploid segregant, named 16D, could be isolated that showed a similar phenotype in terms of acetic acid tolerance.

### Example 2: QTL mapping with pooled F1 segregants

Mapping the genetic determinants that are responsible for the high acetic acid tolerance of 16D was initiated by crossing the haploid segregants ER18 and 16D. The resulting hybrid strain was subsequently sporulated to obtain segregants that contain a mixture of the parental genomes. Obtained segregants were subsequently screened for high acetic acid tolerance, resulting in the identification of 27 (out of 288) segregants that were able to ferment glucose in the presence of 0.9 % acetic acid, which is comparable with the tolerance observed for the superior parent strain. These 27 segregants were therefore selected for pooled-segregant whole-genome sequencing analysis. Genomic DNA isolated from the two parent strains, a pool of the 27 selected segregants and a control pool of 27 randomly selected segregants was sent for custom sequencing analysis using the Illumina HiSeq2000 technology (BGI, Hong Kong, China). The sequence reads from parent strains ER18 and 16D were aligned with the reference sequence from strain S288C. A total number of 23,150 SNPs between ER18 and 16D could be identified, which were subsequently filtered according to the method described by Duitama et al. (2012). The SNP variant frequencies were calculated by dividing the number of the alternative variant by the total number of aligned reads. The calculated variant frequencies were subsequently plotted against the respective chromosomal positions. The underlying structure in the SNP variant frequencies scatterplot of a given chromosome was identified by fitting smoothing splines in the generalized linear mixed model framework, as described by Claesen et al. (2013). Variant frequencies that significantly deviate from 50% (random segregation) are indicative of genetic linkage to the phenotype.

The results from the QTL mapping show two loci on the genome with a strong linkage to the superior segregant 16D: QTL1 on chromosome XIII and a second QTL on chromosome XVI. The statistical significance of QTL1 was confirmed using the Hidden Markov Model described previously, stretching from position 181019 - 294166. Both QTLs were further investigated by scoring selected SNPs in the 27 individual segregants in order to precisely determine the SNP variant frequencies and the statistical significance of the genetic linkage. Using a binomial test previously described (Swinnen et al., 2012; Claessen et al., 2013), both loci were found to be statistically significant. Furthermore, the size of both QTLs could be decreased to regions stretching from roughly 224000 - 277000 for QTL1 on chromosome XIII, and 568000 - 615000 for QTL2 on chromosome XVI.

*GLO1* was confirmed as causative gene for acetic acid tolerance by RHA

### Example 3: Fermentation assay of RHA strains

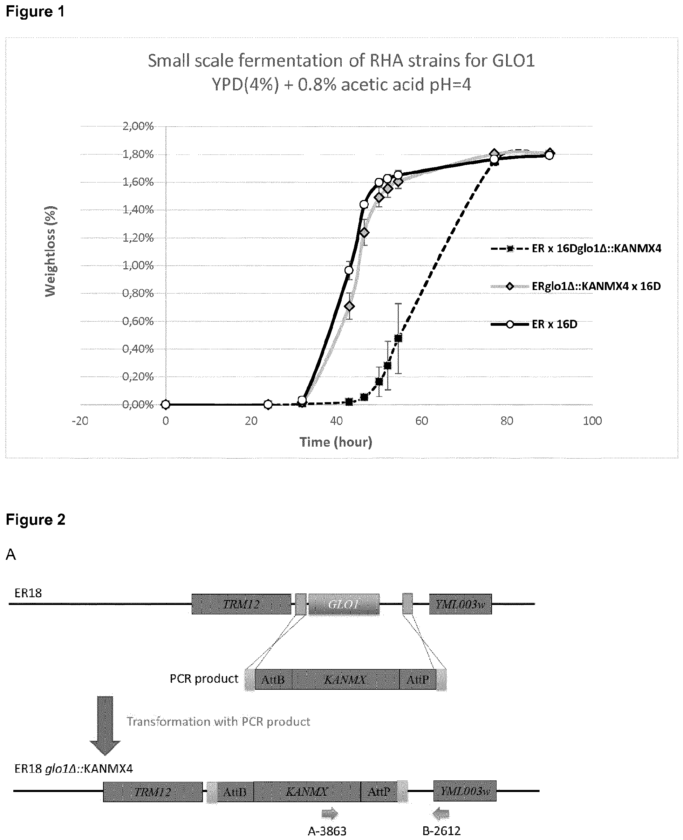

Figure 1 shows the fermentation profiles of the RHA strains for *GLO1*. Every point represents the average of two biological repeats. The error bars indicate the standard error of the mean.

The strains with at least one allele originating from the 16D strain show superior fermentation performance in presence of acetic acid.

### Example 4: Replacement of the GLO1 allele from strain ER18 byt the allele from stain 16D

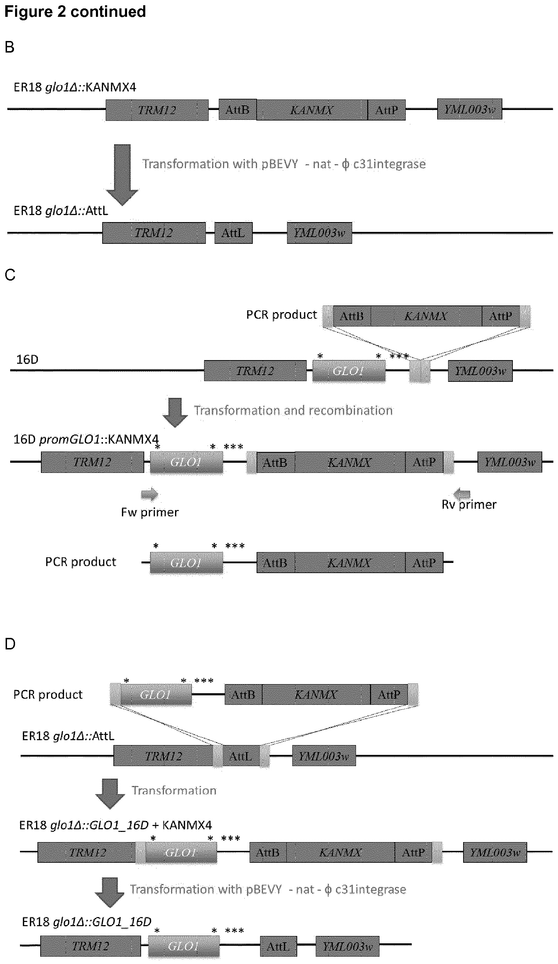

In order to upgrade the *GLO1* allele of ER18, a fragment comprising the ORF, 631 bp upstream and 44bp downstream of the ORF of *GLO1* was replaced by its 16D counterpart. The method to replace the allele comprises three steps:
1. Deletion of the region containing the ORF of *GLO1,* 631 bp upstream and 44 bp downstream in ER18. Primers B-2610 and B-2609 are used to amplify the deletion cassette from plasmid pJET1,2-AttB-KANMX-AttP.
   Both primers contain a 19bp region, binding to pJET1,2-B-KANMX-P and 50bp tails that are homologous to the nucleotides flanking the region that needs to be deleted. In the schematic representation of Figure 2A, these homologous regions are shown as light grey boxes. After transformation, colonies will be selected on YPD plates containing geneticin (200mg/l). Hereafter, colonies were confirmed by PCR with primer couple A-3863/B-2612.
   Hereafter, strain ER18 *glo1Δ::KANMX4* was transformed with plasmid pBEVY-nat-Phic31integrase. After selection on YPD plates containing nourseothricin (100mg/l), the kanamycin marker was removed due to the action of the phage derived phiC31 integrase, leaving an AttL sequence at the recombination site (Figure 2B).
   After confirming of the loss of the KANMX marker by checking the lack of growth on YPD geneticin plates, the strain was cured of the plasmid by growing several rounds in liquid YPD medium.
2. Next, 16D was tagged by a kanamycin marker, 631 bp upstream of *GLO1*. As in step 1, primers were used that contain a 19bp region binding to pJET1,2-B-KANMX-P and 50bp tails that are homologous to the regions flanking the location where the marker needs to be inserted. The primers used for amplification of the cassette from pJET1,2-B-KANMX-P are B-2610 and B-2827.
   After transformation of this fragment, selection on YPD plates containing geneticin and confirmation of the colonies by PCR with primer couple A-3863/B-2612, genomic DNA of this strain was used as a template for amplification of the tagged *GLO1_16D* allele. Primers B-2965 and B-2611 were used for amplification of the tagged *GLO1_16D* allele. (Figure 2C)
3. Finally, the PCR product of the tagged *GLO1_16D* allele, containing the *GLO1* allele of 16D linked to a KANMX cassette, was transformed in ER18 glo1Δ::AttL, the strain obtained after step 1. After transformation of this fragment, selection on YPD plates containing geneticin and confirmation of the colonies by PCR with primer couple A-3863/B-2612, the KANMX cassette is removed by the action of the phiC31 integrase (described previously). (Figure 2D)

### Example 5: The GLO1 allele from strain 16D is needed and sufficient to confer acetic acid tolerance

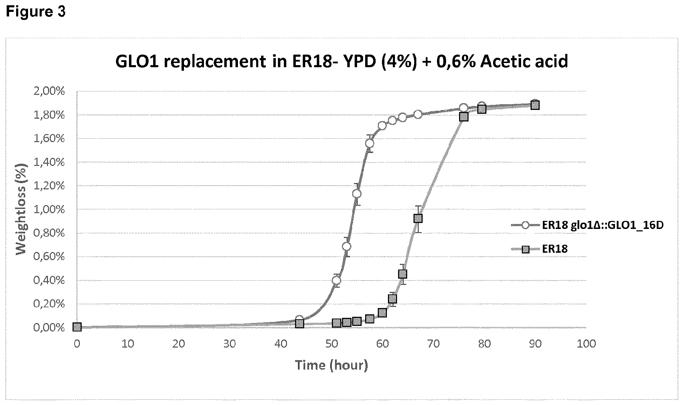

The fermentation profiles of the ER18 parental strain, and the ER18 strain in which the original *GLO1* allele has been replaced by an 16D *GLO1* allele are shown in Figure 3. Every point represents the average of two biological repeats. The error bars indicate the standard error of the mean. ER18 is the original inferior parent. ER18 *glo1Δ::GLO1_16D* is the ER18 strain in which the *GLO1* gene comprising the ORF, 631 bp upstream and 44 bp downstream of the ORF of *GLO1* were replaced by its 16D counterpart.

**Table II. Presence of non-synonymous mutations and the corresponding codons and encoded amino acids in GLO1 from different S. cerevisiae strains for which the whole genome sequence is available.**

| ***GLO1*** | | | | |
|---|---|---|---|---|
| | nt (+106-108) | aa (36) | nt (+964-966) | aa (322) |
| **ER18** | ACC | T | CAT | H |
| **16D** | GC**T** | A | TAT | Y |
| **S288C** | ACC | T | CAT | H |
| **AWRI1631** | GC**T** | A | TAT | Y |
| **AWRI796** | GC**T** | A | TAT | Y |
| **BY4741** | ACC | T | CAT | H |
| **BY4742** | ACC | T | CAT | H |
| **CBS7960** | GC**T** | A | CAT | H |
| **CEN.PK113** | ACC | T | CAT | H |
| **CLIB215** | GC**T** | A | TAT | Y |
| **EC1118** | GC**T** | A | TAT | Y |
| **EC9-8** | GC**T** | A | TAT | Y |
| **FL100** | ACC | T | CAT | H |
| **FostersB** | GC**T** | A | CAT | H |
| **FostersO** | GC**T** | A | CAT | H |
| **JAY291** | GC**T** | A | CAT | H |
| **Kyokai7** | ACC | T | CAT | H |
| **LavinQA23** | GC**T** | A | ----- | - |
| **PW5** | AC**T** | T | CAT | H |
| **RM11-1a** | GC**T** | A | TAT | Y |
| **Sigma1278b** | ACC | T | CAT | H |
| **T7** | AC**T** | T | CAT | H |
| **UC5** | ACC | T | CAT | H |
| **VL3** | ----- | - | TAT | Y |
| **Vin13** | GC**T** | A | TAT | Y |
| **W303** | ACC | T | CAT | H |
| **YJM269** | ACC | T | CAT | H |
| **YJM789** | AC**T** | T | CAT | H |
| **ZTW1** | ACC | T | TAT | Y |

*GLO1* of strain LalvinQA23 has an early stop codon resulting in a truncated ORF that lacks amongst others nt 964-965 and codes for a truncated protein that lacks amongst others aa 322.

*GLO1* of strain VL3 lacks nucleotides 1 - 558 of the ORF and starts with the ATG at position 559-561 resulting in a shortened protein. Hence, it lacks nt 106 - 108 and aa 36, but not nt 964 - 966 and aa 322.

**Table III: GLO1 Promoter mutations: comparison of strains**

| | -782 | -775 | -645b | -645a | -562 | -559 | -531 | -460 | -431 | -385b | -385a | -384 | -273 | -230 | -219 | -135 | -77 | -64 | -48 |
|---|---|---|---|---|---|---|---|---|---|---|---|---|---|---|---|---|---|---|---|
| **ER18** | A | A | - | - | T | G | C | C | A | A | T | T | C | A | C | C | - | G | T |
| **16D** | G | G | C | A | C | A | C | T | G | A | T | T | T | T | T | G | A | G | - |
| **S288C** | A | G | - | - | C | G | T | T | G | - | - | C | C | A | C | C | A | A | T |
| **AWRI1631** | G | G | C | A | C | A | C | T | G | A | T | T | T | T | T | G | A | G | - |
| **AWRI796** | G | G | C | A | C | A | C | T | G | A | T | T | T | T | T | G | A | G | T |
| **CBS7960** | G | G | C | A | C | A | C | T | G | A | T | T | T | A | C | G | A | G | - |
| **CEN.PK113** | A | G | - | - | C | G | T | T | G | - | - | C | C | A | C | C | A | A | T |
| **CLIB215** | G | G | C | A | C | A | C | T | G | A | T | T | T | T | T | G | A | G | - |
| **EC1118** | G | G | C | A | C | A | C | T | G | A | T | T | T | T | T | G | A | G | - |
| **EC9-8** | G | G | C | A | C | A | C | T | G | A | T | T | T | T | T | G | A | G | - |
| **FL100** | A | G | - | - | C | A | C | T | G | A | T | T | T | T | T | G | A | A | T |
| **FostersB** | G | G | C | A | C | A | C | T | G | A | T | T | T | W* | Y** | G | A | G | - |
| **FostersO** | G | G | C | A | C | A | C | T | G | A | T | T | T | W* | Y** | G | A | G | - |
| **JAY291** | G | G | C | A | C | A | C | T | G | A | T | T | T | A | C | G | A | G | - |
| **Kyokai7** | A | A | - | - | T | G | C | C | G | A | T | T | C | A | C | C | A | G | T |
| **LalvinQA23** | G | G | C | A | C | A | C | T | G | A | T | T | T | T | T | G | A | G | - |
| **PW5** | A | G | - | - | C | A | C | T | G | A | T | T | T | A | C | G | A | G | - |
| **RM11-1a** | G | G | C | A | C | A | C | T | G | A | T | T | T | T | T | G | A | G | - |
| **Sigma1278b** | A | G | - | - | C | G | T | T | G | - | - | C | C | A | C | C | A | A | T |
| **T7** | A | G | - | - | C | A | C | T | G | A | T | T | T | A | C | G | A | G | - |
| **UC5** | A | A | - | - | T | G | C | C | G | A | T | T | C | A | C | C | A | G | T |
| **VL3** | G | G | C | A | C | A | C | T | G | A | T | T | T | T | T | G | A | G | - |
| **Vin13** | G | G | C | A | C | A | C | T | G | A | T | T | T | T | T | G | A | G | - |
| **W303** | A | G | - | - | C | G | T | T | G | - | - | C | C | A | C | C | A | A | T |
| **YJM269** | A | G | - | - | C | G | T | T | G | - | - | C | C | A | C | C | A | G | T |
| **YJM789** | A | G | - | - | C | A | C | T | G | A | T | T | T | A | C | G | A | G | - |
| **ZTW1** | A | A | - | - | T | G | C | C | G | A | T | T | C | A | C | C | A | G | T |
| | | | | | | | | | | | | | | | | | | | |
| * W : A, T or U | | | | | | | | | | | | | | | | | | | |
| ** Y: C, T or U | | | | | | | | | | | | | | | | | | | |

### REFERENCES

- Abbott, D.A. et al. (2007). Generic and specific transcriptional responses to different weak organic acids in anaerobic chemostat cultures of Saccharomyces cerevisiae. Fems Yeast Research 7, 819-833.
- Almeida, J.R.M. et al. (2007). Increased tolerance and conversion of inhibitors in lignocellulosic hydrolysates by Saccharomyces cerevisiae. Journal og Chemical Technology and Biotechnology 82, 340-349.
- Bellissimi, E., van Dijken, J.P., Pronk, J.T. & van Maris, A.J.A. (2009). Effects of acetic acid on the kinetics of xylose fermentation by an engineered, xylose-isomerase-based Saccharomyces cerevisiae strain. Fems Yeast Research 9, 358-364.
- Casey, E., Sedlak, M., Ho, N.W. & Mosier, N.S. (2010). Effect of acetic acid and pH on the cofermentation of glucose and xylose to ethanol by a genetically engineered strain of Saccharomyces cerevisiae. Fems Yeast Research 10, 385-393.
- Deutschbauer, A.M. & Davis, R.W. (2005). Quantitative trait loci mapped to single-nucleotide resolution in yeast. Nat Genet 37, 1333-1340.
- Ehrenreich, I.M. et al. (2010). Dissection of genetically complex traits with extremely large pools of yeast segregants. Nature 464, 1039-1042.
- Flint, J. & Mott, R. (2001). Finding the molecular basis of quantitative traits: successes and pitfalls. Nat Rev Genet 2, 437-445.
- Gietz, R.D., Schiestl, R.H., Willems, A.R. & Woods, R.A. (1995). Studies on the transformation of intact yeast cells by the LiAc/SS-DNA/PEG procedure. Yeast 11, 355-360.
- Glazier, A.M., Nadeau, J.H. & Aitman, T.J. (2002). Finding genes that underlie complex traits. Science 298, 2345-2349.
- Hasunuma, T. et al. (2011). Metabolic pathway engineering based on metabolomics confers acetic and formic acid tolerance to a recombinant xylose-fermenting strain of Saccharomyces cerevisiae. Microb Cell Fact 10, 2.
- Huang, H. et al. (2011). Identification of crucial yeast inhibitors in bio-ethanol and improvement of fermentation at high pH and high total solids. Bioresour Technol 102, 7486-7493.
- Huxley C, Green ED, Dunham I. 1990. Rapid assessment of S. cerevisiae mating type by PCR. Trends Genet 6: 236.
- Koppram, R., Albers, E. & Olsson, L. (2012). Evolutionary engineering strategies to enhance tolerance of xylose utilizing recombinant yeast to inhibitors derived from spruce biomass. Biotechnol Biofuels 5, 32.
- Li, B.Z. & Yuan, Y.J. (2010).Transcriptome shifts in response to furfural and acetic acid in Saccharomyces cerevisiae. Appl Microbiol Biotechnol 86, 1915-1924.
- Limtong, S., Sumpradit, T., Kitpreechavanich, V., Tuntirungkij, M., Seki, T. and Yoshida, T. (2000). Effect of Acetic acid on growth and ethanol fermentation of xylose fermenting yeasts and Saccharomyces cerevisiae.Kastaert J. (Nat. Sci) 34, 64-73.
- Matsushika, A. & Sawayama, S. (2012). Characterization of a Recombinant Flocculent Saccharomyces cerevisiae Strain That Co-Ferments Glucose and Xylose: II. Influence of pH and Acetic Acid on Ethanol Production. Appl Biochem Biotechnol.
- Mira, N.P., Palma, M., Guerreiro, J.F. & Sa-Correia, I. (2010a). Genome-wide identification of Saccharomyces cerevisiae genes required for tolerance to acetic acid. Microb Cell Fact 9, 79-91.
- Mira, N.P., Becker, J.D. & Sa-Correia, I. (2010b). Genomic expression program involving the Haa1p-regulon in Saccharomyces cerevisiae response to acetic acid. OMICS 14, 587-601.
- Narendranath, N.V., Thomas, K.C. & Ingledew, W.M. (2001). Effects of acetic acid and lactic acid on the growth of Saccharomyces cerevisiae in a minimal medium. J Ind Microbiol Biotechnol 26, 171-177.
- Olsson, L. and Hahn-Hägerdal, B. (1993). Fermentative performance of bacteria and yeast in lignocellulose hydrolysates. Process Biochem. 28, 249-257.
- Parts, L. et al. (2011). Revealing the genetic structure of a trait by sequencing a population under selection. Genome Res.
- Sherman, F. & Hicks, J. (1991). Micromanipulation and dissection of asci. Methods Enzymol 194, 21-37.
- Steinmetz, L.M. et al. (2002). Dissecting the architecture of a quantitative trait locus in yeast. Nature 416, 326-330.
- Swinnen, S. et al. (2012). Identification of novel causative genes determining the complex trait of high ethanol tolerance in yeast using pooled-segregant whole-genome sequence analysis. Genome Res 22, 975-984.
- Taherzadeh, M.J., Niklasson, C. & Lidén, G. (1997). Acetic acid - friend of foe in anaerobic batch conversion of glucose to ethanol by Saccharomyces cerevisiae. Chemical Engineering Science 52, 2653-2659.
- Tanaka, K., Ishii, Y., Ogawa, J. And Shima, J. (2012). Enhancement of acetic acid tolerance in Saccharomyces cerevisiae by overexpression of the HAA1 gene, encoding a transcription factor. Appl. Environ. Microbiol. 78, 8161-8163.
- Winzeler EA, Shoemaker DD, Astromoff A, Liang H, Anderson K, Andre B, Bangham R, Benito R, Boeke JD, Bussey H, et al. 1999. Functional characterization of the S. cerevisiae genome by gene deletion and parallel analysis. Science 285: 901-906.
- Wright, J. et al. (2011). Batch and continuous culture-based selection strategies for acetic acid tolerance in xylose-fermenting Saccharomyces cerevisiae. Fems Yeast Research.
- Zhang, J.G. et al. (2011). Improvement of acetic acid tolerance and fermentation performance of Saccharomyces cerevisiae by disruption of the FPS1 aquaglyceroporin gene. Biotechnol Lett 33, 277-284.

## Claims

1. A xylose fermenting yeast strain that is mutated or genetically engineered to produce ethanol on the base of xylose, comprising a GLO1 allele, which confers to the strain an improved fermentation performance in the presence of at least 0.4% acetic acid in the medium as compared to a control strain with an identical background except for the GLO1 allele, and wherein the improved fermentation performance is at least one of faster fermentation rate and a shorter lag period, wherein said GLO1 allele is an allele encoding SEQ ID NO: 2 or an allele consisting of SEQ ID NO: 1.

2. A xylose fermenting yeast strain according to claim 1, wherein the GLO1 allele confers to the strain an improved fermentation performance in the presence of at least 0.5%, 0.6%, 0.7% or 0.8% acetic acid in the medium as compared to a control strain with an identical background except for the GLO1 allele.

3. A xylose fermenting yeast strain according to claim 1 or 2, wherein the GLO1 allele is overexpressed by at least one of incorporating more than one copy of the allele in the strain, or by placing the coding sequence of the allele under control of a strong promoter.

4. A xylose fermenting yeast strain according to any one of claims 1-3, wherein the yeast strain is selected from the group consisting of *Saccharomyces* sp., *Pichia* sp., *Candida* sp. *Pachysolen* sp. and *Spathaspora* sp.

5. A xylose fermenting yeast strain according to claim 4, wherein the yeast strain is a *Saccharomyces cerevisiae.*

6. A process for producing bioethanol, comprising a step wherein a yeast strain according to any one of claims 1-5 produces ethanol on the base of xylose.

7. A process according to claim 6, wherein a hydrolysate of lignocellulose is the source for the production of bioethanol.

8. Use of GLO1 for obtaining acetic acid tolerance in yeast.

9. The use of GLO1 according to claim 8, wherein said GLO1 is an allele encoding SEQ ID NO: 2.

10. The use of GLO1 according to claim 9, wherein said GLO1 consists of SEQ ID NO: 1.

11. The use according to any of claims 8-10, wherein said use is replacement of an original GLO1 allele by the GLO1 allele encoding SEQ ID NO: 2.

12. The use according to any of claims 8-11, wherein said use is an overexpression.

13. The use according to any of claims 8-12, wherein said yeast is a *Saccharomyces* species.

14. A method to obtain an acetic acid tolerant yeast, comprising the replacement of an original GLO1 allele by the GLO1 allele encoding SEQ ID NO: 2.

15. A method for selecting an acetic acid tolerant yeast, comprising the identification of the presence of the GLO1 allele encoding SEQ ID NO: 2.

## Patentansprüche

1. Xylose fermentierender Hefestamm, der mutiert oder gentechnisch verändert ist, um Ethanol auf der Basis von Xylose zu produzieren, umfassend ein GLO1-Allel, das dem Stamm eine verbesserte Fermentationsleistung in Gegenwart von mindestens 0,4 % Essigsäure in dem Medium im Vergleich zu einem Kontrollstamm mit einem identischen Hintergrund mit Ausnahme von dem GLO1-Allel verleiht, und wobei die verbesserte Fermentationsleistung mindestens eine von einer schnelleren Fermentationsrate und einer kürzeren Verzögerungsperiode ist, wobei das GLO1-Allel ein Allel, das SEQ ID Nr. 2 kodiert, oder ein Allel, das aus SEQ ID Nr. 1 besteht, ist.

2. Xylose fermentierender Hefestamm nach Anspruch 1, wobei das GLO1-Allel dem Stamm eine verbesserte Fermentationsleistung in Gegenwart von mindestens 0,5 %, 0,6 %, 0,7 % oder 0,8 % Essigsäure in dem Medium im Vergleich zu einem Kontrollstamm mit einem identischen Hintergrund mit Ausnahme von dem GLO1-Allel verleiht.

3. Xylose fermentierender Hefestamm nach Anspruch 1 oder 2, wobei das GLO1-Allel durch mindestens eines von einem Einbinden von mehr als einer Kopie des Allels in den Stamm oder Unterstellen der kodierenden Sequenz des Allels der Kontrolle eines starken Promotors überexprimiert wird.

4. Xylose fermentierender Hefestamm nach einem der Ansprüche 1-3, wobei der Hefestamm aus der Gruppe bestehend aus *Saccharomyces* sp., *Pichia* sp., *Candida* sp., *Pachysolen* sp. und *Spathaspora* sp. ausgewählt ist.

5. Xylose fermentierender Hefestamm nach Anspruch 4, wobei der Hefestamm eine *Saccharomyces cerevisiae* ist.

6. Verfahren zum Produzieren von Bioethanol, das einen Schritt umfasst, wobei ein Hefestamm nach einem der Ansprüche 1-5 Ethanol auf der Basis von Xylose produziert.

7. Verfahren nach Anspruch 6, wobei ein Hydrolysat von Lignocellulose die Quelle für die Produktion von Bioethanol ist.

8. Verwendung von GLO1 zum Erhalten von Essigsäuretoleranz in Hefe.

9. Verwendung von GLO1 nach Anspruch 8, wobei die GLO1 ein Allel, das SEQ ID Nr. 2 kodiert, ist.

10. Verwendung von GLO1 nach Anspruch 9, wobei die GLO1 ein Allel, das aus SEQ ID Nr. 1 besteht, ist.

11. Verwendung nach einem der Ansprüche 8-10, wobei die Verwendung ein Austausch eines Ursprungs-GLO1-Allels durch das GLO1-Allel, das SEQ ID Nr. 2 kodiert, ist.

12. Verfahren nach einem der Ansprüche 8-11, wobei die Verwendung eine Überexpression ist.

13. Verfahren nach einem der Ansprüche 8-12, wobei die Hefe eine *Saccharomyces-*Spezies ist.

14. Verfahren zum Erhalten einer essigsäuretoleranten Hefe, das den Austausch eines Ursprungs-GLO1-Allels durch das GLO1-Allel, das SEQ ID Nr. 2 kodiert, umfasst.

15. Verfahren zum Auswählen einer essigsäuretoleranten Hefe, das die Identifizierung des Vorliegens des GLO1-Allels, das SEQ ID Nr. 2 kodiert, umfasst.

## Revendications

1. Souche de levure à fermentation de xylose qui est mutée ou créée par génie génétique pour produire de l'éthanol sur la base de xylose, comprenant un allèle GLO1 qui confère à la souche une performance de fermentation améliorée en présence d'au moins 0,4 % d'acide acétique dans le milieu par rapport à une souche témoin avec un contexte identique mis à part l'allèle GLO1, et dans laquelle la performance de fermentation améliorée est au moins un élément parmi une vitesse de fermentation plus rapide et une période de latence plus courte, dans laquelle ledit allèle GLO1 est un allèle codant pour SEQ ID N° : 2 ou un allèle constitué de SEQ ID N° : 1.

2. Souche de levure à fermentation de xylose selon la revendication 1, dans laquelle l'allèle GLO1 confère à la souche une performance de fermentation améliorée en présence d'au moins 0,5 %, 0,6 %, 0,7 % ou 0,8 % d'acide acétique dans le milieu par rapport à une souche témoin avec un contexte identique mis à part l'allèle GLO1.

3. Souche de levure à fermentation de xylose selon la revendication 1 ou 2, dans laquelle l'allèle GLO1 est surexprimé par au moins une action parmi l'incorporation de plus d'une copie de l'allèle dans la souche, ou par le placement de la séquence codante de l'allèle sous le contrôle d'un promoteur fort.

4. Souche de levure à fermentation de xylose selon l'une quelconque des revendications 1 à 3, dans laquelle la souche de levure est sélectionnée parmi le groupe constitué de *Saccharomyces* sp., *Pichia* sp., *Candida* sp., *Pachysolen* sp. et *Spathaspora* sp.

5. Souche de levure à fermentation de xylose selon la revendication 4, dans laquelle la souche de levure est une *Saccharomyces cerevisiae.*

6. Procédé de production de bioéthanol, comprenant une étape dans laquelle une souche de levure selon l'une quelconque des revendications 1 à 5 produit de l'éthanol sur la base de xylose.

7. Procédé selon la revendication 6, dans lequel un hydrolysat de lignocellulose est la source pour la production de bioéthanol.

8. Utilisation de GLO1 pour obtenir une tolérance à l'acide acétique dans de la levure.

9. Utilisation de GLO1 selon la revendication 8, dans laquelle ledit GLO1 est un allèle codant pour SEQ ID N° : 2.

10. Utilisation de GLO1 selon la revendication 9, dans laquelle ledit GLO1 est constitué de SEQ ID N° : 1.

11. Utilisation selon l'une quelconque des revendications 8 à 10, dans laquelle ladite utilisation est le remplacement d'un allèle GLO1 d'origine par l'allèle GLO1 codant pour SEQ ID N° : 2.

12. Utilisation selon l'une quelconque des revendications 8 à 11, dans laquelle ladite utilisation est une surexpression.

13. Utilisation selon l'une quelconque des revendications 8 à 12, dans laquelle ladite levure est une espèce de *Saccharomyces.*

14. Procédé d'obtention d'une levure tolérante à l'acide acétique, comprenant le remplacement d'un allèle GLO1 d'origine par l'allèle GLO1 codant pour SEQ ID N° : 2.

15. Procédé de sélection d'une levure tolérante à l'acide acétique, comprenant l'identification de la présence de l'allèle GLO1 codant pour SEQ ID N° : 2.
